# EUROPÄISCHE PATENTSCHRIFT

(11) **EP 1 501 445 B1**
(45) Veröffentlichungstag und Bekanntmachung des Hinweises auf die Patenterteilung: **11.07.2007**
(21) Anmeldenummer: 03725155.0
(22) Anmeldetag: 06.05.2003
(51) Int. Cl.: A61F 2/00, A61F 2/02

(54) **FLACHIGES IMPLANTAT AUS TEXTILEM FADENMATERIAL, INSBESONDERE HERNIENNETZ**
FLAT IMPLANT MADE OF TEXTILE THREAD MATERIAL, PARTICULARLY A HERNIA MESH
IMPLANT PLAT EN MATIERE TEXTILE, EN PARTICULIER FILET HERNIAIRE

(30) Priorität: 07.05.2002 DE 10221320
(43) Veröffentlichungstag der Anmeldung: 02.02.2005
(73) Patentinhaber: GfE Medizintechnik GmbH, 90431 Nürnberg (DE)
(72) Erfinder: FRICKE, Helmut, 38536 Meinersen-Ahnsen (DE); BUTTSTÄDT, Johannes, 90574 Rosstal (DE)
(74) Vertreter: Hübner, Gerd
(86) Internationale Anmeldenummer: PCT/EP2003/004732
(87) Internationale Veröffentlichungsnummer: WO 2003/094781

(56) Entgegenhaltungen:
- EP-A- 1 099 421
- WO-A-02/22047
- US-A- 4 839 215

## Beschreibung

Die Erfindung betrifft ein flächiges Implantat aus textilem Fadenmaterial, insbesondere ein Herniennetz.

Ein flächiges Implantat in einer zweischichtigen Ausführungsform ist aus der EP 1 099 421 A1 bekannt. Beide Schichten bestehen aus einem Gewebe oder Gewirke aus Polymerfilamenten beispielsweise aus Polyester, Polypropylen oder Polytetrafluorethylen. Das Flächengewicht einer Schicht ist dabei mit bis zu 50 g/m² angegeben.

Aus der US-A-4,839,215 ist ein flächiges Implantat auf der Basis eines Netzes mit Webstruktur entnehmbar, bei dem biokompatible Partikel in Form von Hülsen beispielsweise aus Titan perlschnurartig auf den Netzfaden aufgefädelt sind. Das gezeigte Implantat ist offensichtlich für die mikroinvasive Chirurgie nicht geeignet.

Etwa 3 % aller Einwohner eines Landes sind im Laufe Ihres Lebens von einem Leistenbruch betroffen. Die Operation eines Leistenbruches gehört zu den weltweit am häufigsten durchgeführten chirurgischen Eingriffen. Dabei werden über 75 % der Patienten mit Operationstechniken behandelt, die den Verschluss der Bauchlücke mit einem Kunststoffnetz, einem sogenannten Herniennetz, vornehmen.

Um einen dauerhaften Erfolg einer Hernienversorgung mit einem Kunststoffnetz zu gewährleisten, sollte das implantierte Netz den folgenden Anforderungen gerecht werden:
- chemisch inert
- keine physikalischen Veränderungen im Kontakt mit Körperflüssigkeiten
- keine Auslösung von Entzündungen und Fremdkörperreaktionen
- nicht cancerogen
- nicht allergen
- Widerstandsfähig gegenüber mechanischen Belastungen
- sterilisierbar und
- herstellbar in der benötigten Form.

Als Grundmaterial für Herniennetze werden in der modernen Chirurgie hauptsächlich die folgenden drei Kunststoffmaterialien eingesetzt:
- Polyester (PET)
- Polytetrafluorethylen (PTFE) und
- Polypropylen (PP).

Das Netzdesign unterscheidet sich darüber hinaus in der Web- bzw. Wirkart. Es kommen monofile und multifile sowie vliesartige Netze zum Einsatz, die sich zudem in ihrer Maschengröße und ihrem Flächengewicht unterscheiden. Ein großer Teil der durchgeführten Hernienoperationen mit Einsatz eines Kunststoffnetzes führt zu klinisch zufriedenstellenden Ergebnissen. Die Rezidivraten belaufen sich auf weniger als 10 %. Immer wieder wird jedoch auch von Komplikationen aufgrund der Implantation von Kunststoffnetzen berichtet. Es besteht deshalb ein Bedarf, ein Netz mit optimalen Trageigenschaften für den Patienten zu schaffen. Dabei ist die entscheidende Anforderung an Neuentwicklungen, ein Netz zu entwickeln, das die entzündlichen Reaktionen auf ein Minimum verringert bzw. die Körperverträglichkeit des Netzes auf ein Maximum erhöht.

Die Erfindung besteht darin, dass das flächige Implantat aus textilem Fadenmaterial, insbesondere Herniennetz, in Form eines einschichtigen Netzes mit einem Flächengewicht von 5 bis 40 g/m² und mit einer Oberflächenmetallisierung insbesondere enthaltend Titan vorliegt.

Durch die starke Verringerung des Flächengewichtes des Netzes bzw. des Implantates ergeben sich zahlreiche Vorteile für die Handhabung und Einheilung des Implantates. Zunächst wird die Menge an Fremdkörper, die implantiert wird, verringert, was zu einer Verminderung der Reizung des Bindegewebes und zu einer Verringerung von Abwehrreaktionen führt. Durch die geringe MateriaImenge ist es auch außerordentlich flexibel, so dass es sich leicht an Oberflächen des Körpers anschmiegt und dann auch in der einmal eingenommenen Lage und Stellung verbleibt.

Das Netz besitzt wie oben bereits angegeben weiterhin mit Vorteil eine Metallisierung an der Oberfläche, wobei eine solche Metallisierung, die titanhaltig ist, bevorzugt ist. Solche Metallisierungen unter Anwendung eines PACVD-Verfahrens sind beispielsweise aus der DE 199 45 299 A bekannt. Eine Oberflächenmetallisierung der Fäden, insbesondere mit Titan bringt eine gute Gewehefreundlichkeit.

Das Flächengewicht des erfindungsgemäßen Netzes liegt mit Vorteil bei 10 bis 39 g/m², bevorzugt bis 37 g/m². Die Fäden sind vorzugsweise Monofilamente. Das Netz kann in verschiedenen Ausführungsformen vorliegen. Bei einer besonders bevorzugten Ausführungsform der Erfindung ist das Netz aus einzeln geführten Monofilamenten gefertigt- Bei diesen Ausführungsformen liegt das Flächengewicht bevorzugt bei 10 bis 20 g/m², insbesondere bei 16 g/m² bzw. - bei stärkerer Auslegung des Monofilaments - bei 32 bis 39 g/m². Bei einer anderen Ausführungsform der Erfindung ist das Netz aus doppelt geführten Monofilamenten gefertigt. Hierbei liegt das Flächengewicht insbesondere bei 20 bis 37 g/m², wobei etwa 35 bis 37 g/m² die Regel sind.

Auch die Fadenstärke ist gering. Sie liegt vorzugsweise im Bereich von 10 bis 150 µm, insbesondere zwischen 30 und 80 µm. Als besonders vorteilhaft haben sich Fadenstärken im Bereich von 70 bis 75 µm, insbesondere 60 bis 70 µm erwiesen. Die Feinheit liegt bei etwa 8 bis 70 dtex, insbesondere ca. 30 dtex. Aber auch mit einer Fadenstärke von 90 µm entsprechend 58 dtex lassen sich erfolgreich einsetzbare Implantate herstellen.

Das Netz ist mit Vorteil ein Gewebe, ein Gewirk oder Gestrick, wobei ein Gewirk, insbesondere ein Kettengewirk, bevorzugt ist. Besonders günstig sind durchbrochene oder undurchbrochene Gewirkkonstruktionen, insbesondere solche, die durchsichtig sind. Zur Herstellung eignen sich Kettenwirkmaschinen mit ein oder zwei Legeschienen. Bevorzugte Bindungsarten sind die folgenden Legungen: Atlas, Tüll, Mesh und Ajour. Die Maschendichte kann in weiten Grenzen variieren und liegt mit Vorteil bei 2500 bis 25000, insbesondere 4000 bis 14000 Maschen pro dm², bevorzugter bis 12000 Maschen pro dm². In der Praxis hat sich eine Maschendichte zwischen 5000 und 11000, insbesondere zwischen 5000 und 7000 als günstig erwiesen. Die Maschenfeinheit liegt vorteilhaft bei E 8 bis E 44 (Nadeln pro inch).

Das erfindungsgemäße Netz ist hochporös. Seine Porosität liegt mit Vorteil im Bereich von 65 bis 85 %, insbesondere im Bereich von 70 bis 80 %. Eine Porosität von ca. 73 bis 75 % hat sich in der Praxis als vorteilhaft gezeigt. Weiterhin ist das Netz mit Vorteil durchsichtig, was sich in Bezug auf seine Platzierung während der Operation als vorteilhaft erweist. Es besitzt relativ große lichte Maschenöffnungen in der Größe von 0,3 bis 2 mm², insbesondere 0,5 bis 1,5 mm². Bewährt haben sich lichte Maschenöffnungen von ca. 1 mm². Eine Gitterstruktur des Netzes ist besonders vorteilhaft. Bei besonders bevorzugten Ausführungsformen haben die Maschenöffnungen die Form von Quadraten oder Parallelogrammen, die von dünnen Gitterstegen eingerahmt sind. Die Bindung des Netzes ist mit besonderem Vorteil eine Atlas-Bindung. Die Fäden im Bereich der Gitterstege weisen Schlaufen auf, wobei sich bei einfach gebundenen monofilen Fäden eine Stärke der Gitterstege von 3 Fadenstärken ergibt. Werden zwei parallele monofile Fäden verwendet, dann ergibt sich eine Stärke der Gitterstege von jeweils 6 Fäden, wie sich aus der nachfolgenden Beschreibung von Ausführungsformen zeigt.

Als Material für die erfindungsgemäßen Implantate eignet sich besonders ein nicht resorbierbarer inerter Kunststoff, wobei Polypropylen bevorzugt ist. Das erfindungsgemäße Implantat besteht mit Vorteil ausschließlich aus nicht resorbierbarem Material. Es ist weiterhin, abgesehen von einer Metallisierung, mit Vorteil frei von einer Imprägnierung oder Beschichtung.

Die Dicke des erfindungsgemäßen Netzes bzw. Implantates liegt bevorzugt im Bereich von 0,10 bis 0,40 mm, insbesondere 0,15 bis 0,30 mm. Eine Dicke von ca. 0,20 mm ist besonders vorteilhaft.

In Versuchen hat sich gezeigt, dass das erfindungsgemäße Netz bei laparoskopischer Operationstechnik in einfacher Weise an die Bauchwandinnenseite angelegt werden kann und dort aufgrund seines geringen Gewichtes haften bleibt. Dies wird noch unterstützt durch die insbesondere aus einer Titanlegierung bestehende Oberflächenmetallisierung, die für eine Hydrophilierung des Netzes sorgt. Durch die damit verbesserte Benetzbarkeit saugt sich das Herniennetz gewissermaßen an das zu stützende Gewebe, was für ein bereitwilligeres Auffalten des laparoskopisch eingebrachten Netzes sorgt. Ein Verrutschen oder eine Faltenbildung beim Wiederverschließen der laparoskopisch präparierten Kavität ist damit nicht zu befürchten. Damit ist insgesamt die Netzplatzierung leichter möglich als bei vergleichbaren schweren Netzkonstruktionen. Durch die geringe Materialmenge, die mit dem erfindungsgemäßen Netz eingebracht wird, und insbesondere durch die leichte, dünne, grobmaschige und flexible Gestaltung des Netzes ergeben sich hervorragende Langzeitergebnisse.

Weitere Merkmale, Einzelheiten und Vorteile der Erfindung ergeben sich aus der nachfolgenden Beschreibung, in der Ausführungsbeispiele leichter Herniennetze anhand der beigefügten Zeichnungen näher erläutert werden. Es zeigen:
- Fig. 1 bis 3: ausschnittsweise vergrößerte Darstellungen von leichten Herniennetzen in drei unterschiedlichen Ausführungsformen,
- Fig. 4 und 5: ausschnittsweise vergrößerte Darstellungen der Herniennetze gemäß Figur 1 und 2 im Bereich deren Schnittkante, sowie
- Fig. 6: eine Umrissdarstellung eines Herniennetzes.

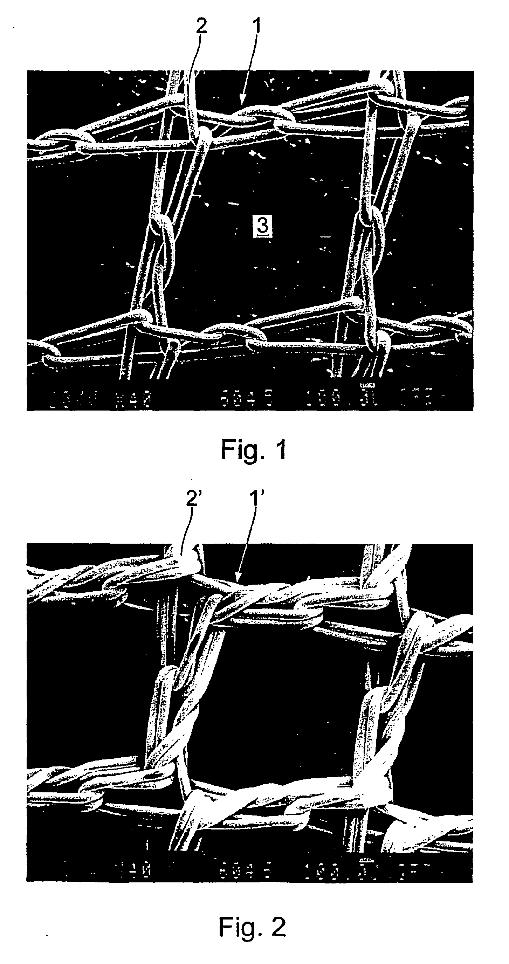

Bei der in der Zeichnung in Figur 1 dargestellten Ausführungsform der Erfindung ist ein Ausschnitt eines Herniennetzes 1 mit 40-facher Vergrößerung dargestellt. Monofile Polypropylenfäden 2 mit einer Dicke von 66 µm sind auf einer Kettenwirkmaschine in zweireihiger Atlaslegung zu einem grobmaschigen Netz gewirkt. Durch diese Wirkart ist ein Gitter entstanden, das parallelogrammartige Öffnungen 3 besitzt, wobei der spitze Winkel des Parallelogramms etwa bei ca. 80° liegt. Die Öffnungsweite der Öffnungen 3 beträgt ca. 1mm. In der Gitterstruktur laufen die Einzelfäden 2 unter Schlaufenbildung immer wieder zurück, so dass die Einzelfäden 2 an jeder Stelle der Gitterstege 3-fach liegen. Das Netz 1 besitzt ca. 10800 Maschen pro dm². Das Flächengewicht des Netzes beträgt 16 g/m². Die Reißfestigkeit des Netzes liegt deutlich über der Reißfestigkeit des zu stützenden Gewebes (ca. 16 N/cm). Die Höchstzugkraft in Anlehnung an DIN 53857 beträgt ≥ 50 N in der Länge und ≥ 40 N in der Breite. Die Höchstzugkraftdehnung liegt bei ≥ 20 % in der Länge und ≥ 40 % in der Breite.

Die Ränder des auf die gewünschte Größe von etwa 10 x 10 cm bis 30 x 30 cm zugeschnittenen Netzes 1 können thermisch oder durch Klebemittel abgebunden sein. Die Porosität des Netzes liegt bei 80 %. Es ist gut durchsichtig. Das Netz ist einschichtig aufgebaut. Es besitzt eine Dicke von ca. 0,20 mm und ist sehr flexibel. Das Netz lässt sich bei der Operation leicht an die Bauchwandinnenseite des Patienten anlegen und haftet dort von selbst. Die Oberfläche des Netzes ist durch Plasmabeschichtung (PACVD) mit einer Titanschicht metallisiert, die so dünn ist, dass das Metall mit dem Auge nicht erkennbar ist. Aufgrund der geringen Materialmenge, der geringen Oberfläche der Kunststofffäden und der Gewebefreundlichkeit der Oberfläche des Polypropylens durch die Metallisierung zeigt das Netz ein sehr gutes Einwachsverhalten und dauerhaft gute Ergebnisse.

Das Netz 1' der in Figur 2 dargestellten Ausführungsform ist aus dem gleichen Fadenmaterial hergestellt und besitzt die gleiche Wirkbindung wie die Ausführungsform nach Figur 1. Das Netz ist ebenfalls mit Titan metallisiert. Im Unterschied zu Figur 1 sind jedoch zwei monofile Fäden als Doppelfaden 2' parallel geführt. Dadurch besitzt das Netz 1' ein etwa doppel so großes Flächengewicht von 37 g/m². Die Gitterstruktur ist im wesentlichen quadratisch. Die Bindung ist wiederum Atlas. Durch die Parallelführung zweier monofiler Fäden liegt die Fadenzahl an jeder Stelle der Gitterstruktur bei sechs. Die lichte Maschenweite des Netzes 1' ist trotz der Verdopplung der Fäden etwa gleich groß wie bei der Ausführungsform nach Figur 1. Die Maschenzahl liegt bei etwa 10000 Maschen pro dm². Die Porosität des Netzes liegt bei 73 %. Die Dicke des Netzes beträgt 0,25 mm. Die Höchstzugkraft in Anlehnung an DIN 53857 liegt in Längsrichtung bei ≥ 200 N in der Länge und bei ≥ 100 N in der Breite. Die Höchstzugkraftdehnung liegt bei ≥ 3 0 % in der Länge und bei ≥ 60 % in der Breite.

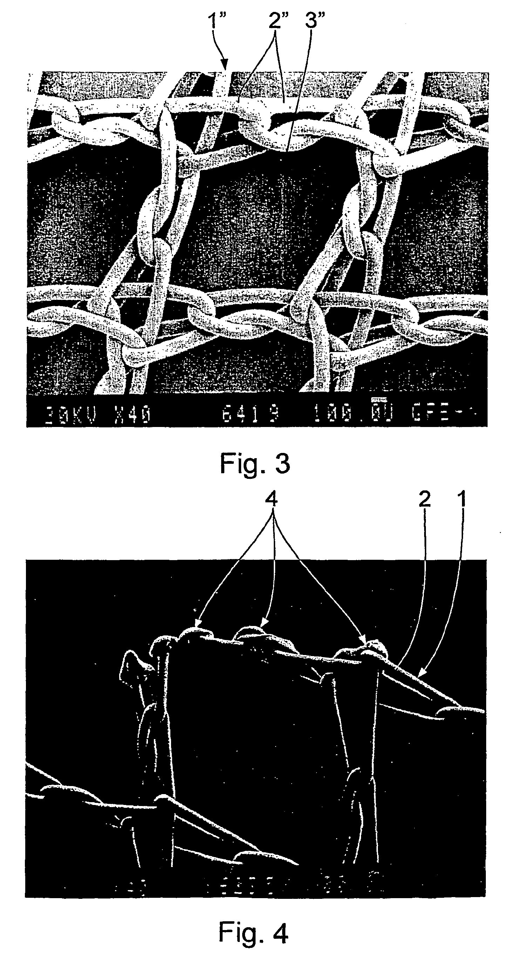

Das Netz 1" der in Figur 3 dargestellten Ausführungsform besitzt die gleiche Wirkbindung wie die Ausführungsform nach Figur 1 und zeigt ebenfalls eine einfache Filamentführung. Allerdings findet ein dickerer Faden 2" mit einer Stärke von 0,090 mm (58 dtex) Verwendung, sodass ein Netz geschaffen wird, das in Eigenschaften und Maschenkonstruktion mit dem schweren Herniennetz gemäß Figur 2 vergleichbar ist. Das Flächengewicht des Netzes 1" beträgt insoweit 32 bis 39 g/m², seine Dicke liegt bei 0,3 mm. Bei der verwendeten Atlas-Bindung des Netzgewirkes liegt die Maschenzahl in der Größenordnung von 8300 bis 13300 Maschen pro dm². Die Werte für die Höchstzugkraft und Höchstzugkraftdehnung bei dem Netz 1 " gemäß Figur 3 entsprechen denen des in Figur 2 dargestellten Netzes 1'. Die optische Porosität auf Grund der Öffnungen 3" liegt bei 70 %.

Die Netze nach Figur 2 und 3 sind zwar schwerer als das Netz nach Figur 1, aber immer noch leicht genug, um ein hervorragendes Einwachsverhalten zu zeigen. Bei der Operation faltet sich das Netz leicht auf und legt sich an die Bauchwandinnenseite an, ohne später Verschiebungen oder Faltenbildung zu zeigen. Langzeitversuche bei Tieren ergaben hervorragende Ergebnisse.

Eine weitere Problematik bei Herniennetzen liegt darin, dass diese aus einer breiteren, endlosen Gewirkebahn herausgeschnitten werden. Bei einem üblichen Stanzen bilden sich freie Fadenenden, sodass sich Fäden aus dem Gewirkeverbund lösen können.

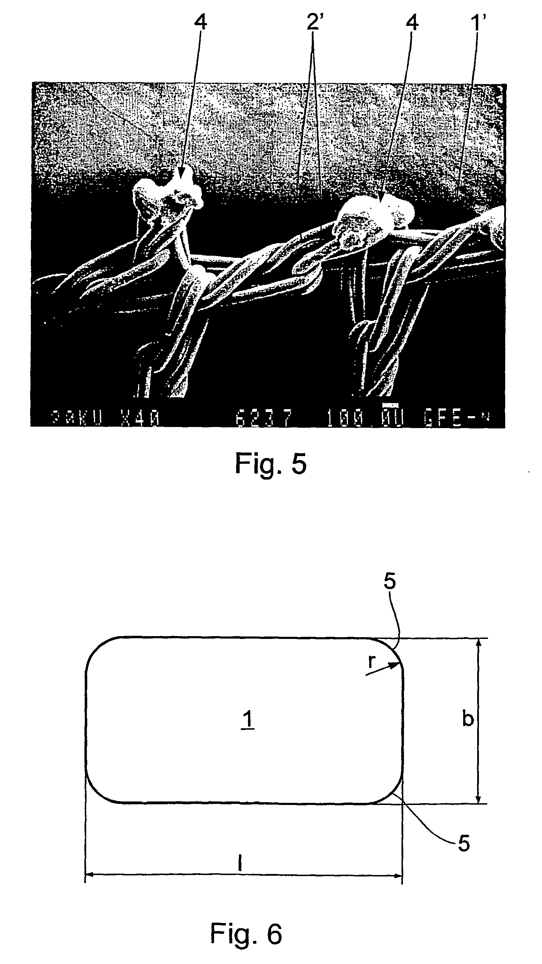

Um dies zu vermeiden, erfolgt der Netzzuschnitt vorteilhafterweise durch Laserschneiden. Dabei werden - wie aus den Figuren 4 und 5 deutlich wird - die Schnittenden 4 der Einzelfäden 2, 2' miteinander verschmolzen, sodass das geschilderte Herauslösen von freien Fäden unterbunden wird. Der Laserzuschnitt erhöht damit die Netzqualität.

Wie ferner aus Figur 6 hervorgeht, wird der Zuschnitt eines Herniennetzes 1 so angelegt, dass die Netzecken 5 abgerundet sind. Der Rundungsradius r liegt dabei in einem Bereich von 4 bis 40 mm, bevorzugter Wert liegt bei r = 22 mm. Durch die abgerundeten Ecken wird eine Irritation des Körpergewebes nach der Netzimplantation aufgrund mechanischer Reizung durch spitze Ecken vermieden.

Die Umrissdimensionen des Herniennetzes 1 liegen bei einer Länge 1 von etwa 15 cm und einer Breite b von etwa 10 cm.

## Patentansprüche

1. Flächiges Implantat aus textilem Fadenmaterial, insbesondere Herniennetz, in Form eines einschichtigen Netzes (1;1'; 1") mit einem Flächengewicht von 5 bis 40 g/m², **dadurch gekennzeichnet, dass** das Netz (1;1'; 1") eine Oberflächenmetallisierung aufweist, insbesondere eine solche, die Titan enthält.

2. Implantat nach Anspruch 1, **dadurch gekennzeichnet, dass** sein Flächengewicht bei 10 bis 39 g/m², insbesondere bis 37 g/m² liegt.

3. Implantat nach einem der vorhergehenden Ansprüche, **dadurch gekennzeichnet, dass** das Netz (1, 1") aus einem einzelgeführten Monofilament (2; 2") gefertigt ist und das Flächengewicht insbesondere bei 10 bis 20 g/m² oder 32 bis 39 g/m² liegt.

4. Implantat nach Anspruch 1 oder 2, **dadurch gekennzeichnet, dass** das Netz (1') aus einem doppelgeführten Monofilament (2') gefertigt ist und das Flächengewicht insbesondere bei 20 bis 37 g/m² liegt.

5. Implantat nach einem der vorhergehenden Ansprüche, **dadurch gekennzeichnet, dass** die Fäden (2; 2'; 2") eine Fadenstärke von 10 bis 150 µm, insbesondere 30 bis 80 µm oder etwa 90 µm besitzen.

6. Implantat nach einem der vorhergehenden Ansprüche, **dadurch gekennzeichnet, dass** das Netz (1; 1'; 1") ein Gewirk oder ein Gestrick ist, insbesondere ein Gewirk.

7. Implantat nach einem der vorhergehenden Ansprüche, **dadurch gekennzeichnet, dass** das Netz (1; 1') eine Maschendichte von 2500 bis 25000 Maschen pro dm², insbesondere 4000 bis 14000 Maschen pro dm², bevorzugter bis 12000 Maschen pro dm² aufweist.

8. Implantat nach einem der vorhergehenden Ansprüche, **dadurch gekennzeichnet, dass** das Netz (1; 1'; 1'') eine Porosität von 65 bis 85 %, insbesondere von 70 bis 80 % besitzt.

9. Implantat nach einem der vorhergehenden Ansprüche, **dadurch gekennzeichnet, dass** das Netz (1; 1'; 1") durchsichtig ist.

10. Implantat nach einem der vorhergehenden Ansprüche, **dadurch gekennzeichnet, dass** das Netz eine Gitterstruktur besitzt, wobei Öffnungen (3; 3'; 3") der Gitterstruktur vorzugsweise quadratisch oder parallelogrammartig ausgebildet sind.

11. Implantat nach einem der vorhergehenden Ansprüche, **dadurch gekennzeichnet, dass** das Netz lichte Maschenöffnungen (3; 3'; 3") in der Größe von 0,3 bis 2 mm², insbesondere 0,5 bis 1,5 mm², besitzt.

12. Implantat nach einem der vorhergehenden Ansprüche, **dadurch gekennzeichnet, dass** das Netz (1; 1'; 1") in Atlas-Legung gewirkt ist.

13. Implantat nach einem der vorhergehenden Ansprüche, **dadurch gekennzeichnet, dass** das Netz (1; 1'; 1") vollständig aus nichtresorbierbarem Material, insbesondere Polypropylen, besteht.

14. Implantat nach einem der vorhergehenden Ansprüche, **dadurch gekennzeichnet, dass** das Netz (1; 1'; 1") aus monofilen Fäden besteht.

15. Implantat nach einem der vorhergehenden Ansprüche, **dadurch gekennzeichnet, dass** das Netz (1; 1'; 1") eine Dicke von 0,10 bis 0,40 mm, insbesondere 0,15 bis 0,30 mm, besitzt.

16. Implantat nach einem der vorhergehenden Ansprüche, **dadurch gekennzeichnet, dass** das einschichtige Netz (1; 1'; 1") in einer rechteckigen Grundform mit abgerundeten Ecken (5) vorliegt, deren Rundungsradius (r) zwischen 4 und 40 mm, vorzugsweise bei 22 mm liegt.

17. Implantat nach einem der vorhergehenden Ansprüche, **dadurch gekennzeichnet, dass** die Kanten (4) des einschichtigen Netzes (1; 1') laserbeschnitten sind.

## Claims

1. A flat implant of textile thread material, in particular a hernia mesh implant, in the form of single-layered mesh fabric (1; 1'; 1") of a basis weight of 5 to 40 g/m², **characterized in that** the mesh fabric (1; 1', 1") includes a surface metallization, in particular containing titanium.

2. An implant according to claim 1, **characterized in that** its basis weight amounts to 10 to 39 g/m², in particular to 37 g/m².

3. An implant according to one of the preceding claims, **characterized in that** the mesh fabric (1, 1") is made from an individually supplied monofilament (2; 2") and that the basis weight amounts in particular to 10 to 20 g/m² or 32 to 39 g/m².

4. An implant according to claim 1 to 2, **characterized in that** the mesh fabric (1') is made from a doubly supplied monofilament (2') and that the basis weight amounts in particular to 20 to 37 g/m².

5. An implant according to one of the preceding claims, **characterized in that** the threads (2; 2', 2") have a gauge of 10 to 150 µm, in particular 30 to 80 µm or approximately 90 µm.

6. An implant according to one of the preceding claims, **characterized in that** the mesh fabric (1; 1'; 1") is hosiery or knit fabric, in particular hosiery.

7. An implant according to one of the preceding claims, **characterized in that** the mesh fabric (1; 1') has a mesh density of 2,500 to 25,000 meshes per square decimeter, in particular 4,000 to 14,000 meshes per square decimeter, more preferably of 12,000 meshes per square decimeter.

8. An implant according to one of the preceding claims, **characterized in that** the mesh fabric (1; 1'; 1") has a porosity of 65 to 85 %, in particular of 70 to 80 %.

9. An implant according to one of the preceding claims, **characterized in that** the mesh fabric (1; 1'; 1") is transparent.

10. An implant according to one of the preceding claims, **characterized in that** the mesh fabric has a lattice structure, the apertures (3; 3', 3") of the lattice structure preferably being of the type of squares or parallelograms.

11. An implant according to one of the preceding claims, **characterized in that** the mesh fabric includes clear mesh apertures (3; 3'; 3") of a size of 0.3 to 2 mm², in particular 0.5 to 1.5 mm².

12. An implant according to one of the preceding claims, **characterized in that** the mesh fabric (1; 1'; 1'') has a warp satin texture.

13. An implant according to one of the preceding claims, **characterized in that** the mesh fabric (1; 1'; 1") entirely consists of non-resorbable material, in particular polypropylene.

14. An implant according to one of the preceding claims, **characterized in that** the mesh fabric (1; 1'; 1") is comprised of monofil threads.

15. An implant according to one of the preceding claims, **characterized in that** the mesh fabric (1; 1'; 1") has a thickness of 0.10 to 0.40 mm, in particular 0.15 to 0.30 mm.

16. An implant according to one of the preceding claims, **characterized in that** the single-layered mesh fabric (1; 1', 1") has a rectangular basic shape with rounded corners (5), the radius of curvature (r) of which ranges between 4 and 40 mm, preferably being 22 mm.

17. An implant according to one of the preceding claims, **characterized in that** the edges (4) of the single-layered mesh fabric (1; 1') are laser-cut.

## Revendications

1. Implant plat en matière textile, en particulier filet herniaire, en forme de filet monocouche (1, 1', 1") avec une masse surfacique de 5 à 40 g/m², **caractérisé en ce que** le filet (1, 1', 1") présente une métallisation en surface, en particulier une, contenant du titane.

2. Implant selon la revendication 1, **caractérisé en ce que** sa masse surfacique va de 10 à 39 g/m², en particulier à 37 g/m².

3. Implant selon l'une des revendications précédentes, **caractérisé en ce que** le filet (1, 1") est fabriqué à partir d'un simple monofilament (2, 2") et la masse surfacique est en particulier de 10 à 20 g/m² ou de 32 à 39 g/m².

4. Implant selon la revendication 1 ou 2, **caractérisé en ce que** le filet (1') est fabriqué à partir d'un double monofilament (2') et la masse surfacique est en particulier de 20 à 37 g/m².

5. Implant selon l'une des revendications précédentes, **caractérisé en ce que** les fils (2, 2', 2") ont une force de fil de 10 à 150 µm, en particulier 30 à 80 µm ou environ 90 µm.

6. Implant selon l'une des revendications précédentes, **caractérisé en ce que** le filet (1, 1', 1") est un tissage ou un tricot, en particulier un tissage.

7. Implant selon l'une des revendications précédentes, **caractérisé en ce que** le filet (1, 1') présente une maillage de 2500 à 25000 mailles par dm², en particulier 4000 à 14000 mailles par dm², de préférence jusqu'à 12000 mailles par dm².

8. Implant selon l'une des revendications précédentes, **caractérisé en ce que** le filet (1, 1', 1") possède une porosité de 65 à 85%, en particulier de 70 à 80%.

9. Implant selon l'une des revendications précédentes, **caractérisé en ce que** le filet (1, 1', 1") est transparent.

10. Implant selon l'une des revendications précédentes, **caractérisé en ce que** le filet possède une structure grillagée, où les ouvertures (3, 3', 3") de la structure grillagée sont de préférence carrées ou en parallélogramme.

11. Implant selon l'une des revendications précédentes, **caractérisé en ce que** le filet possède un maillage libre (3, 3',3'') d'une grandeur de 0,3 à 2 mm², en particulier 0,5 à 1,5 mm².

12. Implant selon l'une des revendications précédentes, **caractérisé en ce que** le filet (1, 1', 1") est tissé en jeté atlas.

13. Implant selon l'une des revendications précédentes, **caractérisé en ce que** le filet (1, 1', 1") est constitué entièrement de matière non résorbable, en particulier du polypropylène.

14. Implant selon l'une des revendications précédentes, **caractérisé en ce que** le filet (1, 1', 1") est constitué de fils monofils.

15. Implant selon l'une des revendications précédentes, **caractérisé en ce que** le filet (1, 1', 1") possède une grosseur de 0,10 à 0,40 mm, en particulier 0,15 à 0,30 mm.

16. Implant selon l'une des revendications précédentes, **caractérisé en ce que** le filet monocouche (1, 1', 1") a une forme de base carrée avec des angles arrondis (5), dont le rayon (r) est entre 4 et 40 mm, de préférence de 12 mm.

17. Implant selon l'une des revendications précédentes, **caractérisé en ce que** les bords (4) du filet monocouche (1, 1') sont découpés au laser.
